# EUROPEAN PATENT SPECIFICATION

(11) **EP 3 225 721 B1**
(45) Date of publication and mention of the grant of the patent: **24.07.2019**
(21) Application number: 14907081.5
(22) Date of filing: 26.11.2014
(51) Int. Cl.: C40B 50/06, C40B 50/18, C12N 15/11, C12N 15/10, C12Q 1/68

(54) **METHOD AND REAGENT FOR CONSTRUCTING NUCLEIC ACID DOUBLE-LINKER SINGLE-STRAND CYCLICAL LIBRARY**
VERFAHREN UND REAGENS ZUR KONSTRUKTION EINER ZYKLISCHEN DOPPELLINKER-EINZELSTRANG-NUKLEINSÄUREBIBLIOTHEK
PROCÉDÉ ET RÉACTIF POUR LA CONSTRUCTION D'UNE BANQUE D'ACIDE NUCLÉIQUE SIMPLE BRIN CIRCULAIRE À DOUBLE SÉQUENCE DE LIAISON

(43) Date of publication of application: 04.10.2017
(73) Proprietor: MGI Tech Co., Ltd., Yantian District Shenzhen Guangdong 518083 (CN)
(72) Inventor: JIANG, Yuan, Guangdong 518033 (CN); ZHAO, Xia, Guangdong 518033 (CN); ALEXEEV, Andrei, California 95776 (US); DRMANAC, Radoje, Los Altos Hill, California 94022 (US); ZHANG, Wenwei, Shenzhen Guangdong 518033 (CN); JIANG, Hui, Shenzhen Guangdong 518033 (CN)
(74) Representative: Hamer, Christopher K.
(86) International application number: PCT/CN2014/092297
(87) International publication number: WO 2016/082130

(56) References cited:
- EP-A1- 2 336 354
- EP-A1- 2 565 279
- WO-A1-2012/079486
- WO-A2-01/20039
- WO-A2-2012/106546
- CN-A- 102 016 068
- CN-A- 102 296 065
- CN-A- 102 534 811
- CN-A- 102 628 079
- CN-A- 103 103 624
- CN-A- 103 119 162
- CN-A- 103 290 106
- CN-A- 103 806 111
- RAMOS ROMMEL THIAGO JUCÁ ET AL: "High efficiency application of a mate-paired library from next-generation sequencing to postlight sequencing:Corynebacterium pseudotuberculosisas a case study for microbialde novogenome assembly", JOURNAL OF MICROBIOLOGICAL METHODS, vol. 95, no. 3, 21 June 2013 (2013-06-21), pages 441-447, XP028791347, ISSN: 0167-7012, DOI: 10.1016/J.MIMET.2013.06.006
- KARATA K ET AL: "Construction of a circular single-stranded DNA template containing a defined lesion", DNA REPAIR, ELSEVIER, AMSTERDAM, NL, vol. 8, no. 7, 4 July 2009 (2009-07-04), pages 852-856, XP026192174, ISSN: 1568-7864, DOI: 10.1016/J.DNAREP.2009.03.006 [retrieved on 2009-04-21]
- TATYANA GORBACHEVA ET AL: "Improved transposon-based library preparation for the Ion Torrent platform", BIOTECHNIQUES RAPID DISPATCHES, vol. 58, no. 4, 1 April 2015 (2015-04-01), XP055403825, US ISSN: 0736-6205, DOI: 10.2144/000114277

## Description

### TECHNICAL FIELD

The present invention relates to the field of molecular biology, particularly to a method and a reagent for constructing a library of single-stranded cyclic nucleic acid fragments having double adaptors.

### BACKGROUND OF THE INVENTION

Ever since AB corporation launched the capillary electrophoresis sequencer and the human genome project started in the 1990's, DNA (deoxyribonucleic acid) sequencing technologies have been developing fast at unimaginable speeds. The second and third generations of sequencers were successively launched on the market. Among the second-generation sequencing platforms, the Blackbird sequencing platform from Complete Genomics Corporation (hereinbelow abbreviated as CG) occupies a large market share in clinical research areas such as molecular diagnosis by virtue of its higher sequencing accuracy than other platforms (99.9998%) and greater advantage in sequencing throughput compared to other platforms. However, due to the limitation of sample treatment methods, the library insert fragments of the CG sequencing platform are too small (2×19-35 bp). This results in difficulty in subsequent genomic assembling operations, limiting the application of the platform in genomic de novo sequencing. Moreover, the period of library construction is too long, which would badly retard the progress of scientific research and project operation. The rapid emergence of various next-generation sequencing technologies also poses a challenge. Therefore, it is an urgent and important R&D task to increase the size of library insert fragments, simplify the procedure of library construction, shorten the time required for library construction through technological improvement and innovation, in order to expand the application scope and efficiency of CG sequencing platforms.

The insert fragment introduction process in CG platform's existing construction procedure of a library of single-stranded cyclic nucleic acid fragments having double adaptors considerably restricts the length of library insert fragments. The NT (nick translation) technique used in the SOLiD (Sequencing by Oligonucleotide Ligation and Detection) platform improves the length of library insert fragments to some extent, but has the drawback that the spanning of the insert fragments is very wide. The fragments need to be selected by means of gel recovery, which increases the tediousness of the operation. Moreover, gel recovery would affect the efficiency of recovering the insert fragments.

Additionally, in CG platform's existing construction process of a library of single-stranded cyclic nucleic acid fragments having double adaptors, a plurality of steps of enzymatic reaction and purification are required in order for the sequencing adaptors to be ligated to the genomic DNA fragments to be detected. This takes much time, consumes many reagents and wastes many samples, limiting the speed of sample treatment. Further, the initial sample amount (3 µg) is a disadvantage over other sequencing platforms.

### SUMMARY OF THE INVENTION

The present invention provides a method and a reagent for constructing a library of single-stranded cyclic nucleic acid fragments having double adaptors. The method enables to increase the length of library insert fragments without the need for gel recovery; and the single-stranded nucleic acid molecules can be directly cyclized following denaturation by heat.

In a first aspect of the present invention, there is provided a method for constructing a library of single-stranded cyclic nucleic acid fragments having double adaptors, comprising the following steps:
disrupting a nucleic acid into nucleic acid fragments for library construction;
ligating a first adaptor sequence to both ends of the nucleic acid fragments;
performing a first PCR amplification to obtain first products having the first adaptor sequence at both ends, wherein the primer sequences used in the first PCR have a U base site;
digesting the first products with a USER enzyme to form sticky ends, followed by cyclization to generate a gap; or alternatively, in the case that the primer sequences used in the first PCR also have a nickase recognition sequence, digesting the first products with a USER enzyme to form sticky ends, followed by cyclization, and then followed by digestion of the cyclized products with a nickase to generate a nick;
initiating controlled nick/gap translation reaction from the nick or gap by using the cyclic nucleic acid molecules as template;
digesting and removing the portion of the respective cyclic nucleic acid molecules that does not undergo the controlled nick/gap translation reaction to obtain linear nucleic acid molecules;
ligating a second adaptor sequence to both ends of the linear nucleic acid molecules;
performing a second PCR amplification to obtain second products having the second adaptor sequence at both ends; and
denaturing the second products to obtain single-stranded nucleic acid molecules, and cyclizing one of the single-stranded nucleic acid molecules with a mediating sequence complementary to both ends of the single-stranded nucleic acid molecule to obtain the library of single-stranded cyclic nucleic acid fragments having double adaptors.
In a preferred embodiment of the present invention, the first adaptor sequence comprises a first 5' adaptor sequence and a first 3' L-type adaptor sequence that respectively ligate to the 3' end and the 5' end of each strand of the fragments; the first 5' adaptor sequence comprises a 5'-end-phosphorylated long strand and a complementary short strand, the long strand having a tag sequence in the middle, the short strand having dideoxy modification at the 3' end, and the short strand comprising a U base site; and a portion of the first 3' L-type adaptor sequence which is adjacent to the ligated fragment is complementary to part of the bases of the first 5' adaptor sequence; and
said ligating a first adaptor sequence to both ends of the nucleic acid fragments specifically comprises:
   dephosphorylating the nucleic acid fragments;
   subjecting the dephosphorylated nucleic acid fragments to end repairing;
   ligating the first 5' adaptor sequence to the 3' end of each strand of the nucleic acid fragments;
   digesting the U base site of the short strand of the first 5' adaptor sequence with a USER enzyme;
   phosphorylating the USER enzyme-digested nucleic acid fragments; and
   ligating the first 3' L-type adaptor sequence to the 5' end of each strand of the phosphorylated nucleic acid fragments.

In a preferred embodiment of the present invention, each of the primer sequences used in the first PCR has a nickase recognition sequence and a U base site; and after digesting the U base site with the USER enzyme, sticky ends are formed at both ends of the nucleic acid fragments, and cyclization occurs due to the complementarity of the sticky ends, generating cyclized nucleic acid molecules.

In a preferred embodiment of the present invention, one of the primer sequences used in the first PCR has two U base sites, while the other primer sequence has a U base site; and after digesting the U base sites with the USER enzyme, sticky ends are formed at both ends of the nucleic acid fragments, and cyclization occurs due to the complementarity of the sticky ends, generating cyclized nucleic acid molecules.

In a preferred embodiment of the present invention, following cyclizing the digested first products, the method further comprises: digesting the nucleic acid molecules that are not cyclized.

In a preferred embodiment of the present invention, one of the primer sequences used in the first PCR harbors a biotin label; and prior to the controlled nick/gap translation reaction, streptavidin-labeled magnetic beads are used to bind the products from the first PCR, such that subsequent reactions are performed on the magnetic beads.

In a preferred embodiment of the present invention, in the controlled nick/gap translation reaction, the length of the gap translation fragments generated is controlled by controlling at least one factor selected from the group consisting of the molar ratio of dNTPs to the nucleic acid molecules as template, the enzymatic reaction temperature and the enzymatic reaction time.

In a preferred embodiment of the present invention, said digesting and removing the portion of the respective cyclic nucleic acid molecules that does not undergo the controlled nick/gap translation reaction specifically comprises: first degrading the cyclic nucleic acid molecules with an enzyme having 5'-3' exonuclease activity until the gaps at both ends meet; and then degrading the resulting single strands with an enzyme having 3'-5' exonuclease activity or a single strand exonuclease.

In a preferred embodiment of the present invention, the second adaptor sequence comprises a second 5' adaptor sequence and a second 3' L-type adaptor sequence that respectively ligate to the 3' end and the 5' end of each strand of the linear nucleic acid molecules; the second 5' adaptor sequence comprises a 5-end-phosphorylated long strand and a complementary short strand, the short strand having dideoxy modification at the 3' end, and the short strand comprising a U base site; and a portion of the second 3' L-type adaptor sequence which is adjacent to the ligated fragment is complementary to part of the bases of the second 5' adaptor sequence; and
said ligating a second adaptor sequence to both ends of the linear nucleic acid molecules specifically comprises:
dephosphorylating the linear nucleic acid molecules;
subjecting the dephosphorylated linear nucleic acid molecules to end repairing;
ligating the second 5' adaptor sequence to the 3' end of each strand of the linear nucleic acid molecules;
digesting the U base site of the short strand of the second 5' adaptor sequence with a USER enzyme;
phosphorylating the USER-enzyme digested fragments; and
ligating the second 3' L-type adaptor sequence to the 5' end of each strand of the phosphorylated linear nucleic acid molecules.

In a preferred embodiment of the present invention, following cyclizing the single-stranded nucleic acid molecules, the method further comprises: digesting the single-stranded nucleic acid molecules that are not cyclized.

According to a second aspect of the present invention, there is provided a reagent for constructing a library of single-stranded cyclic nucleic acid fragments having double adaptors, comprising the following components:
a first adaptor sequence, which comprises a first 5' adaptor sequence and a first 3' L-type adaptor sequence that respectively ligate to the 3' end and the 5' end of each strand of the fragments, wherein the first 5' adaptor sequence comprises a 5'-end-phosphorylated long strand and a complementary short strand, the long strand having a tag sequence in the middle, the short strand having dideoxy modification at the 3' end, and the short strand comprising a U base site; and a portion of the first 3' L-type adaptor sequence which is adjacent to the ligated fragment is complementary to part of the bases of the first 5' adaptor sequence;
primers for a first PCR, which have U base sites or have a nickase recognition sequence and a U base site, and which are used to obtain first products having the first adaptor sequence at both ends by the first PCR amplification;
a nickase, which is used for digesting the first products to generate a nick;
a USER enzyme, which is used for digesting the first products to generate sticky ends and gaps for cyclization;
components for gap translation reaction, which are used for initiating controlled nick/gap translation reaction from the nick or gap by using the cyclic nucleic acid molecules as template;
a digestive enzyme, which is used for digesting and removing the portion of the respective cyclic nucleic acid molecules that does not undergo the controlled nick/gap translation reaction to obtain linear nucleic acid molecules;
a second adaptor sequence, which comprises a second 5' adaptor sequence and a second 3' L-type adaptor sequence that respectively ligate to the 3' end and the 5' end of each strand of the linear nucleic acid molecules; wherein the second 5' adaptor sequence comprises a 5-end-phosphorylated long strand and a complementary short strand, the short strand having dideoxy modification at the 3' end, and the short strand comprising a U base site; and a portion of the second 3' L-type adaptor sequence which is adjacent to the ligated fragment is complementary to part of the bases of the second 5' adaptor sequence;
primers for a second PCR, which are used for performing a second PCR amplification to obtain second products having the second adaptor sequence at both ends; and
a mediating sequence, which is complementary to both ends of one of the single-stranded nucleic acid molecules obtained following denaturation of the second products, and which is used for cyclizing the single-stranded nucleic acid molecule to obtain the library of single-stranded cyclic nucleic acid fragments having double adaptors.

In the method for constructing a library of single-stranded cyclic nucleic acid fragments having double adaptors according to the present invention, fragments of particular lengths are generated by means of introduction of a nickase recognition sequence and/or a U base site in the primer sequences for the first PCR, followed by digestion to generate nicks or gaps, and followed by controlled nick/gap translation reaction. This can control the length of the fragments in a certain range while increasing the length of the library insert fragments. Thus, the fragments do not need to be selected by gel recovery. The finally formed single-stranded nucleic acid molecules can be directly cyclized following denaturation by heat without the need for a screening operation, which simplifies the process of library construction. Moreover, the use of the special L-type adaptor sequences for ligation allows for simplifying the steps and shortening the period of operation.

### BRIEF DESCRIPTION OF THE DRAWINGS

Fig. 1 is the flow-process diagram showing the method for constructing a library of single-stranded cyclic nucleic acid fragments having double adaptors according to an embodiment of the present invention;
Fig. 2 is a diagram showing the basic principle underlying the generation of CNT gaps according to an embodiment of the present invention;
Fig. 3 is a diagram showing the basic principle underlying the generation of CNT nicks according to an embodiment of the present invention;
Fig. 4 is a diagram comparing the principle of formation of insert fragments and separation and cyclization of single strands according to a prior art process and the process of the present invention;
Fig. 5 is a diagram comparing a directional adaptor ligation process of the prior art and the L-type adaptor ligation process of the present invention;
Fig. 6 is a schematic depiction of the library construction strategy of controlled nick/gap translation reaction on magnetic beads (CNT on beads) according to an embodiment of the present invention; and
Fig. 7 presents graphs showing the results of Agilent 2100 detection of the PCR products following adaptor B ligation according to an embodiment of the present invention.

### DETAILED DESCRIPTION OF THE INVENTION

The present invention is described in further detail below by reference to particular embodiments. Unless otherwise stated, the techniques used in the following embodiments are all conventional techniques known to a person skilled in the art, and the instruments, equipments and reagents used are all publicly available, e.g. commercially available, to a person skilled in the art.

In the present invention, the concepts of "first" and "second" used in any cases should not be construed as conveying the meaning of order or technique, and they serve only to distinguish the objects to which they refer from other objects.

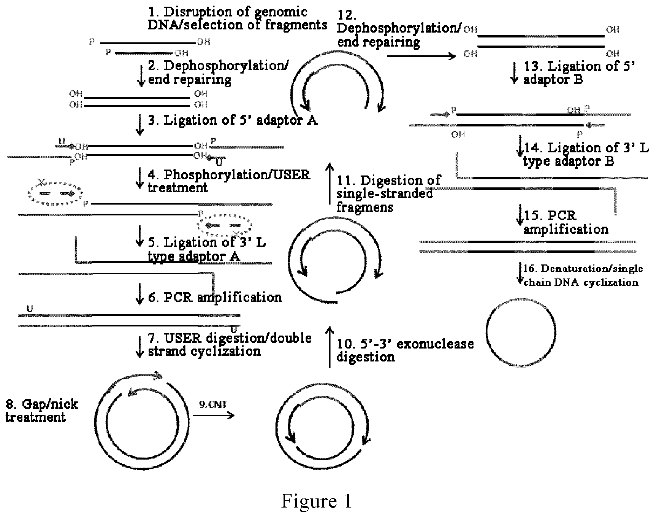

Reference is made to Fig. 1, in which a method for constructing a library of single-stranded cyclic nucleic acid fragments having double adaptors according to an embodiment of the present invention is shown. The method comprises the following steps: disrupting a genomic DNA to form nucleic acid fragments for constructing the library; subjecting the fragments to dephosphorylation and end repairing reactions; ligating a 5' adaptor A sequence to the fragments; subjecting the fragments to USER enzyme digestion and phosphorylation treatment; ligating a 3' L-type adaptor A sequence to the fragments; subjecting the fragments to PCR amplification to obtain products having the 5' adaptor A sequence and the 3' L-type adaptor A sequence at both ends, wherein the sequence of the primers used in the PCR comprises a U base site; digesting the U base site with a USER enzyme to generate gaps, and cyclizing the products following USER enzyme digestion to generate cyclic nucleic acid molecules; initiating controlled nick translation (CNT) from the gaps; degrading the cyclic nucleic acid molecules with an enzyme having 5'-3' exonuclease activity until the gaps at both ends meet, and then degrading the resulting single strands with an enzyme having 3'-5' exonuclease activity or a single strand exonuclease to obtain linear nucleic acid molecules; subjecting the linear nucleic acid molecules to dephosphorylation and end repairing; ligating a 5' adaptor B sequence to the nucleic acid molecules, phosphorylating the nucleic acid molecules, and ligating a 3' L-type adaptor B sequence to the nuclei acid molecules; subjecting the nucleic acid molecules to PCR amplification to obtain products having the 5' adaptor B sequence and the 3' L-type adaptor B sequence at both ends; subjecting the products to denaturation treatment to obtain single-stranded nucleic acid molecules, and cyclizing one of the single-stranded nucleic acid molecules with a mediating sequence complementary to both ends of the single-stranded nucleic acid molecule to obtain the library of single-stranded cyclic nucleic acid fragments having double adaptors.

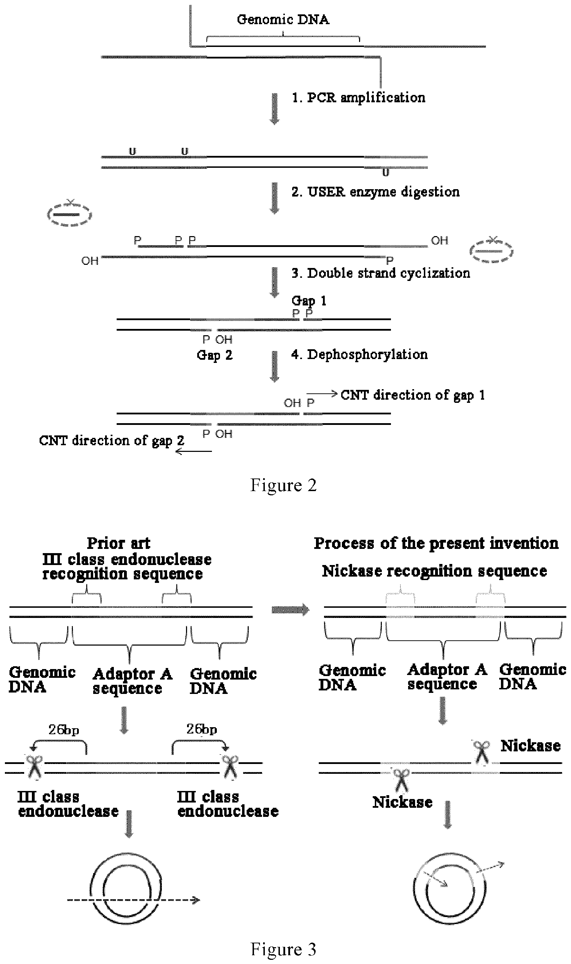

In the method according to the present invention for constructing a library of single-stranded cyclic nucleic acid fragments having double adaptors, as shown in Fig. 1, a U base site is introduced into the primers for the first PCR, and is digested with a USER enzyme to generate a gap as an initiation point for controlled nick translation reaction. The basic principle underlying the generation of the gap is as shown in Fig. 2: (1) after ligating the 5' adaptor A sequence and the 3' L-type adaptor A sequence, the adaptor A ligation products are amplified using primers respectively having two Us and one U; (2) the U bases are digested with a USER enzyme, forming phosphorylated 3' ends and 5' ends at the nicks; (3) performing double strand cyclization by means of the sticky ends generated in the enzyme digestion, wherein after cyclization, the gap on one strand (gap 1, formed by USER enzyme digestion) involves a phosphorylated 3' end and a phosphorylated 5' end, and the gap on the other strand (gap 2, formed due to lack of a matching base at the site after cyclization) involves a dephosphorylated 3'end and a phosphorylated 5'end; and (4) performing dephosphorylation treatment to dephosphorylate the 3' end of gap 1, so as to provide an effective initiation site for CNT.

In the present invention, nicks can also be generated by employing the principle as shown in Fig. 3 and serve as the initiation site of controlled nick translation reaction. In a prior art process, a III class endonuclease recognition sequence is introduced into the adaptor A sequence, and following adaptor A sequence ligation and cyclization, the double strands are digested with a III class endonuclease to generate linear double-stranded DNA; while in a process of the present invention, nickase recognition sequences are introduced into the adaptor A sequence, and following adaptor A sequence ligation and cyclization, each single strand of the cyclized DNA is digested with a nickase (such as Nb.BsrDI, Nb.BsmI, Nt.BbvCI, Nb.Bbv.Nb.BtsI or Nt.BstNBI, etc.) to generate a nick on each single strand, so as to provide an effective initiation site for CNT.

In the present invention, the reaction initiated from the nick or gap is referred to as "controlled nick/gap translation reaction", because the length of the target fragments generated by the reaction can be controlled in a certain range by controlling the usage amount of dNTPs, the usage amount of the nucleic acid molecules as template, the enzyme reaction temperature and time, among other factors. Nucleic acid fragments having a length in a certain range are suitable for a particular sequencing platform. In general, the length of the target fragments in the present invention is preferably controlled in the range of 50-250 bp. Such a length is several times longer than the length of the target fragments obtained by a conventional library construction protocol for a CG sequencing platform. Moreover, the CNT technique of the present application allows for controlling the library insert fragments in a very narrow range without performing gel recovery, which effectively enhances the operability of the gap translation technique.

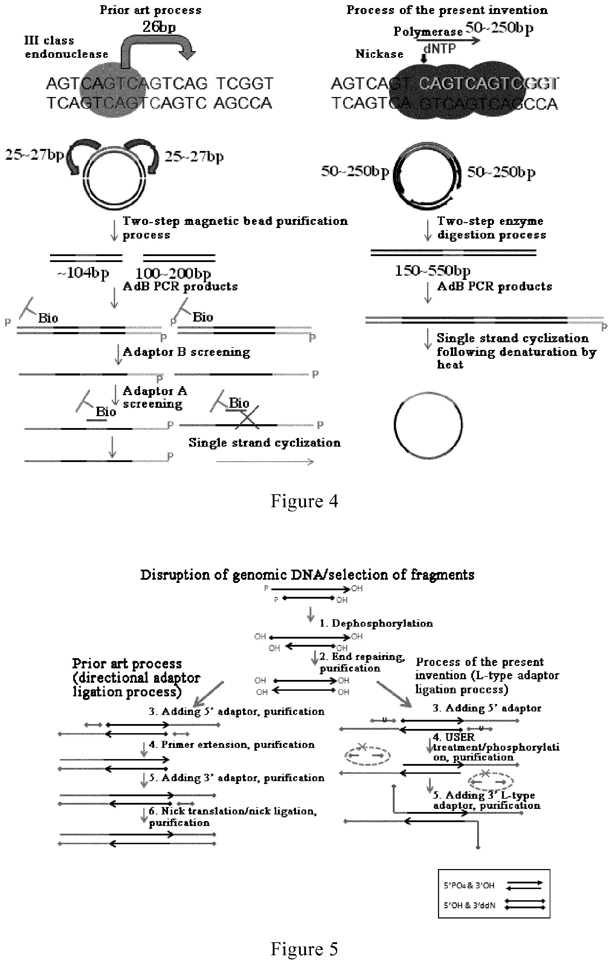

Reference is now made to Fig. 4, in which a prior art process and a process of the present invention are compared. The prior art process takes advantage of the cleaving property of a III class endonuclease, which digests the genomic DNA 25-27 bp from both sides of the adaptor A, forming a target DNA fragment of about 104 bp. Subsequently, the DNA fragments of more than 200 bp not having the adaptor A are removed by means of a two-step magnetic bead purification process. Following this sequence selection with magnetic beads, the digestion products obtained are still mingled with some non-target DNA fragments having main bands of 100-200 bp. Following ligation of the adaptor B, the DNA fragments ligated with the adaptor B are amplified with primers having a biotin-labeled base on one of the primers, wherein the single strands amplified with the primer having a biotin-labeled base thereon are non-target single-stranded nucleic acids. Subsequently, DNA fragments ligated with the adaptor B are enriched using streptavidin magnetic beads, and further, DNA fragments ligated with the adaptor A are enriched by capturing via specific sequence hybridization. Finally, the double-stranded DNA is unwound by means of alkali denaturation to elute the target single-stranded nucleic acids from the streptavidin magnetic beads, and then the target single-stranded nucleic acids are cyclized using a mediating sequence. The whole procedure of the prior art process not only involves tedious steps and lengthy operations, but also consumes expensive reagents (mainly streptavidin magnetic beads). In the process of the present invention, a nick is made using a nickase on the two strands of adaptor A respectively. Then, by taking advantage of the nick translation function of a polymerase in the presence of dNTPs, the nicks are moved from the adaptor A region to either side of the adaptor A. The length through which the nicks are moved can be flexibly controlled by controlling the molar ratio of the dNTPs to the template DNA, the reaction temperature and the reaction time, among other conditions, and the size of the main band of the fragments through which the nicks are moved can be controlled in the range of 50-250 bp. Subsequently, the non-target DNA fragments not having adaptor A are digested by a two-step exonuclease digestion reaction, the remaining fragments being the target DNA fragments having adaptor A, which are subjected to ligation with adaptor B and PCR amplification using primers not having a biotin label. The resulting double-stranded DNAs are unwound simply by high temperature denaturation, and the resulting target single strands are cyclized by means of a mediating sequence, thus separating and cyclizing the target single-stranded DNAs. It can be seen that the single strand cyclization process of the present invention only entails denaturation by heat and hybridization with a mediating sequence for successful separation and cyclization of the target single-stranded nucleic acids. Hence, the process of the present invention not only involves simple steps and easy operations, but also eliminates the need to consume large amounts of expensive reagents, leading to decreased cost in library construction.

In a preferred embodiment of the present invention, ligation is performed using an L-type adaptor instead of a conventional adaptor. Reference is now made to Fig. 5, in which a prior art adaptor ligation process and the adaptor ligation process of the present invention are compared. The prior art process employs a directional adaptor ligation process, whereby directional ligation of the adaptors is ensured and at the same time the problem of inter-ligation of DNA fragments is minimized. The prior art process involves separately designing and stepwise ligating the 5' adaptor and the 3' adaptor. The addition of an adaptor on either end entails synergy of an adaptor sequence, a blocking sequence and a primer sequence. The whole procedure entails 6 enzymatic reaction steps, i.e., dephosphorylation, end repairing, adding 5' adaptor, primer extension, adding 3' adaptor, nick translation and ligation, as well as 5 purification operations, in order to directionally add the sequence of adaptor A to both ends of the target DNA. Such a prior art process suffers from tedious steps, high cost and long period in library construction (the cost of sequences, the cost of enzymatic reaction reagents, and the cost of purification) and high consumption of samples, which does not comply with the requirement of high efficiency and convenience for library construction. On the contrary, the L-type adaptor ligation process of the present invention can increase library construction efficiency and decrease library construction cost while ensuring directional ligation of the adaptors. Although the L-type adaptor ligation process also employs stepwise ligation, the steps involved are simple in comparison to the prior art process. First, a 5' adaptor having a blocking sequence is added. The blocking sequence is 12 bp long and completely complementary to the 5' adaptor, forming a partially complementary double-stranded structure to facilitate ligation of DNA fragments with the 5' adaptor. The blocking sequence has a dideoxy modification at the 3' end and a dephosphorylized base at the 5' end. This not only ensures that the 5' end adaptor is directionally ligated to the 3' end of DNA fragments, but also that the blocking sequence would not ligate to the 5' end of DNA fragments. The blocking sequence has a U base in the middle, such that upon treatment with a USER enzyme, the blocking sequence is "degraded" into two single-stranded DNA fragments of smaller than 8 bp and thus is unwound and detached from the 5' adaptor. Then, an "L" type single-stranded 3' adaptor is added via a process of hybridization followed by ligation. Prior to addition of the L-type adaptor, the 5' end of the DNA fragments needs to be phosphorylated for deblocking. Experiments demonstrated that USER enzyme treatment can be performed concurrently with phosphorylation reaction. Following reaction, purification is conducted with magnetic beads, and the magnetic beads having been washed can be directly resuspended in a ligation reaction buffer for the next step. The L-type adaptor is so ingeniously designed that the last 8 bases at the 3' end thereof are complementary to the last 8 bases at the 5' end of the 5' adaptor, such that the L-type adaptor can directly hybridize to the 5' adaptor. Then, by using a ligase to block the nick, the L-type 3' adaptor is ligated to the 5' end of the DNA fragments. As some of the bases of the L-type adaptor are complementary to some of the bases at the 5' end of the 5' adaptor, while the other bases are not complementary to each other, the adaptor appears an L form, hence called L-type adaptor. After the reaction ends, the ligation products having added with the adaptors can be purified and recovered by further adding a suitable amount of a magnetic bead binding buffer into the magnetic beads. The sequence of adaptor A can be directionally added to both ends of the target DNA relatively fast only through five enzymatic reaction steps, i.e., dephosphorylation, end repairing, adding 5' adaptor, one-step reaction of USER digestion and phosphorylation, adding 5' L type adaptor, as well as three purification operations in the whole procedure. Thus, the steps are simple, the cost of library construction is lowered, and the period of library construction shortened.

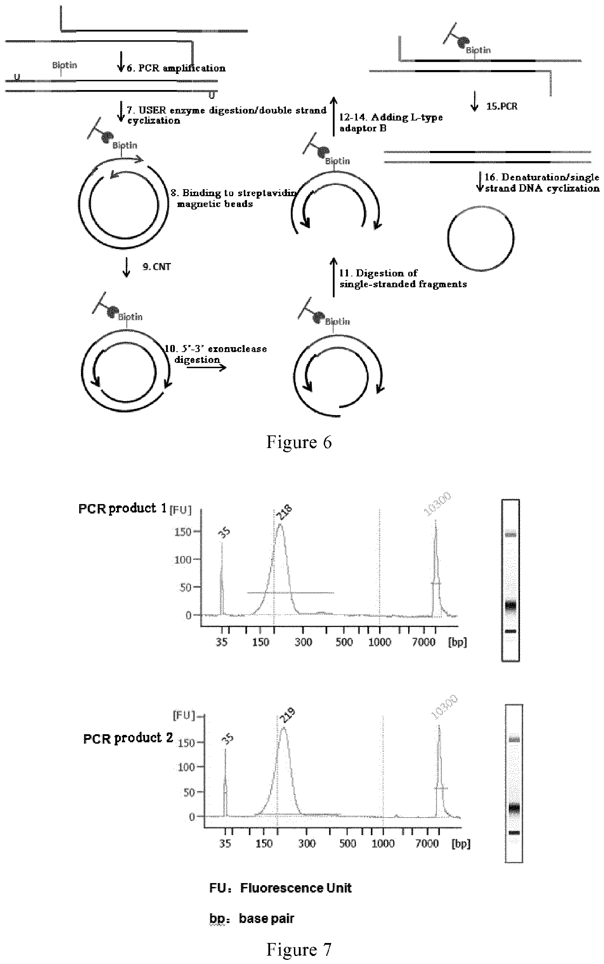

In a preferred embodiment of the present invention, one of the primer sequences used in the first PCR amplification harbors a biotin label. Prior to the controlled nick/gap translation reaction, streptavidin-labeled magnetic beads are used to bind the products from the first PCR amplification, such that subsequent reactions are performed on the magnetic beads. Reference is now made to Fig. 6, in which the on-beads reactions in the present invention are illustrated. Specifically, one of the strands of the products from the first PCR amplification harbors a biotin label, such that the products having been cyclized can bind with streptavidin-labeled magnetic beads. From step 8 to step 14, the enzymatic reaction in each step is performed on the streptavidin-labeled magnetic beads. After reaction, the magnetic beads only need to be adsorbed onto a magnetic rack to remove the reaction solution and then washed to remove the impurities, in order to be ready for the enzymatic reaction in the next step. This eliminates the need for a purification operation following enzymatic reaction in each step of a conventional library construction process, thus saving the reagents for purification. Moreover, the operation time of the magnetic bead washing process is shorter than that of a conventional purification process.

The above analysis shows that, in a preferred embodiment of the present invention, by using the unique CNT technique, L-type adaptor ligation technique, and direct single strand cyclization technique following simple thermal denaturation, improvement and optimization are successfully achieved on the library contruction procedure of the double adaptor library construction process with a CG sequencing platform, such that the size of the library insert fragments is increased 2-10 fold compared with the original size, the whole library construction period and cost are decreased by about 40%, and the initial amount required for library construction is decreased from 3 µg to 500 ng.

The present invention is illustrated in detail with reference to the following example.
1. Disruption of genomic DNA:Genomic DNA can be disrupted in a number of methods, and no matter they are a physical ultrasonic method or an enzymatic reaction method, well-established protocols are commercially available. In this example, the physical ultrasonic method was employed for disruption.
A 96-well PCR plate was added with a polytetrafluoroethylene line. 1 µg of genomic DNA was added, then TE buffer solution or enzyme-free pure water was added to make up to 100 µL. The plate was sealed with a membrane and then placed in an E220 ultrasonic disruptor to conduct ultrasonic disruption. The conditions for disruption were shown in Table 1.

**Table 1**

| Parameter | Value |
|---|---|
| Filling factor | 21% |
| Pressure (PIP) | 500 |
| Pulse factor | 500 |
| Disruption time | 20s, twice |

2. Selection of fragments following disruption: A magnetic bead purification method or gel recovery method can be employed. In this example, the magnetic bead purification method was used.
The disrupted DNA was added with 45 µL of Ampure XP magnetic beads, and the mixture was mixed well and stood for 7-15 min. The resulting mixture was placed on a magnetic rack, and supernatant was collected. The supernatant was added with 18 µL of Ampure XP magnetic beads, and the mixture was mixed well and stood for 7-15 min. The resulting mixture was placed on a magnetic rack, and supernatant was aspirated off. The magnetic beads were washed twice with 75% ethanol and air dried. Then 30 µL of TE solution was added, and the mixture was mixed well and stood for 7-15 min to allow the recovered products to dissolve.
3. Dephosphorylation reaction of the fragments: The recovered products from the previous step were used, and a system was formulated according to Table 2.

**Table 2**

| Reaction component | Volume (µL) |
|---|---|
| 10× NEB buffer 2 | 3.6 |
| Shrimp alkaline phosphatase (1 U/µL) | 3.6 |
| Total | 7.2 |

7.2 µL of the reaction solution was added to the recovered products from the previous step. The mixture was mixed well and incubated at 37°C for 45 min and at 65°C for 10 min. Then the temperature was ramped down to 4°C at a rate of 0.1°C per second.
4. End repairing of the fragments. A system was formulated according to Table 3.

**Table 3**

| Reaction component | Volume (µL) |
|---|---|
| Enzyme-free water | 7.32 |
| 10× NEB buffer 2 | 1.08 |
| 0.1 M adenosine triphosphate | 0.48 |
| 25 mM deoxyribonucleoside triphosphate | 0.48 |
| Bovine serum albumin (10 mg/ml) | 0.24 |
| T4 deoxyribonucleic acid polymerase (3 U/µL) | 1.2 |
| Total | 10.8 |

The system was mixed well and the products from the previous step were added. The mixture was mixed well and incubated at 12°C for 20 min. The reaction was purified with 48 µL of Ampure XP magnetic beads, and the recovered products were dissolved using 40 µL of TE buffer solution.
5. Ligation of 5' adaptor A sequence : The 5' adaptor A sequence used in this example was as follows (in this example, the sequences are shown in the direction of from 5' end to 3'end from left to right, "//" represents modification group, "phos" represents phosphorylation, "dd" represents dideoxy, "bio" represents biotin, and the characters in bold represent a tag sequence).
5' adaptor A sequence: /5phos/ACTGCTGACGTAC**TGTGTCATAAATA**GCACGAGACGTTCTCGACT/3ddC /(SEQ ID NO: 1);
5' blocking sequence: TACGUCAGCAG/3ddT/ (SEQ ID NO: 2).

A mixed solution of 5' adaptor A (10 µM) was formulated according to Table 4.

**Table 4**

| Reaction component | Volume (µL) |
|---|---|
| 5' adaptor A sequence (100 µM) | 12 |
| 5' blocking sequence (100 µM) | 10 |
| TE buffer | 78 |
| Total | 100 |

4.5 µL of the mixed solution of 5' adaptor A formulated (10 µM) was added into the products of the previous step, and the mixture was sufficiently mixed.
A ligation reaction system was formulated according to Table 5.

**Table 5**

| Reaction component | Volume (µL) |
|---|---|
| Enzyme-free pure water | 13.1 |
| 2× ligation buffer 1 | 60 |
| T4 DNA ligase (fast) (600 U/µL) | 2.4 |
| Total | 75.5 |

The 2× ligation buffer 1 used in this example was formulated according to Table 6.

**Table 6**

| Reaction component | Volume (µL) |
|---|---|
| 1 M tromethamine | 37.5 |
| 1 M citric acid | 9.6 |
| 1 M magnesium chloride | 35 |
| 1 M trisodium citrate | 20 |
| 100% glycerol | 50 |
| 10% Tween-80 | 1 |
| 30% polyethylene glycol 8000 | 333 |
| 0.1 M adenosine triphosphate | 10 |
| 0.5 M trichloroethyl phosphate (pH 7.0) | 2 |
| Enzyme-free pure water | 1.9 |
| Total | 500 |

A mixed solution of the ligation reaction system with the adaptor and the products was mixed well, and the mixture was incubated at 25°C for 30 min and at 65°C for 10 min, followed by decreasing the temperature to 4°C.
6. One-step reaction of USER enzyme digestion and phosphorylation: Into the reaction solution from the previous step were added 1.2 µL of USER enzyme (1 U/µL) and 1.2 µL of T4 polynueleotide kinase (10 U/µL). The mixture was mixed well and incubated at 37°C for 20 min. The reaction was purified with 108 µL of Ampure XP magnetic beads. The beads were rinsed with 70% ethanol twice, and with the rinsing liquid having been blot up, were air dried at room temperature for 2 min. Then the beads were resuspended in 48 µL of a 3' L-type adaptor reaction system.
7. Ligation of 3' L-type adaptor A sequence:The 3' L-type adaptor A sequence used in this example was as shown below: CGTTCTCGACUCAGCAGT (SEQ ID NO: 3).
The 3' L-type adaptor reaction system was formulated according to Table 7:

**Table 7**

| Reaction component | Volume (µL) |
|---|---|
| Enzyme-free pure water | 28.98 |
| 3× ligation buffer 2 | 16.02 |
| L-type adaptor sequence (100 µM) | 1.8 |
| T4 DNA ligase (fast) (600 U/µL) | 1.2 |
| Total | 48 |

The Ampure XP magnetic beads resuspended in 48 µL of the 3' L-type adaptor reaction system was incubated in an incubator at a rotation speed of 300 rpm at 25°C for 30 min. After reaction was complete, 43.2 µL of Ampure XP magnetic bead binding buffer was added to conduct incubation at room temperature for 10 min. The supernatant was removed and the beads were washed with 70% ethanol twice, followed by air dried at room temperature for 5-10 min. The recovered products were dissolved with 30 µL of TE buffer solution.
This step achieved the ligation of the target nucleic acid fragments with the adaptor A. The total amount and yield of the products before and after ligation were as shown in Table 8.

**Table 8**

| | Concentration (ng/µL) | Total amount (ng) | Yield |
|---|---|---|---|
| Product 1 | 5.95 | 178.5 | 59.5% |
| Product 2 | 6.11 | 183.3 | 61.1% |
| Product 3 | 5.92 | 177.6 | 59.2% |

8. Polymerase chain reaction:
The sequence of primer 1 was as follows:
   GTCGAGAACGUCTCG/iBiodT/GCTATTTATGACACAGTACGUCAGCAG (SEQ ID NO: 4);
The sequence of primer 2 was as follows:
   ACGTTCTCGACUCAGCAG (SEQ ID NO: 5).

A PCR system was formulated according to Table 9.

**Table 9**

| Reaction component | Volume (µL) |
|---|---|
| Enzyme-free pure water | 186.5 |
| 2× PfuTurbo Cx buffer | 275 |
| PfuTurbo Cx heat-activated nucleic acid polymerase (2.5 U/µL) | 11 |
| 20 µM primer 1 | 13.75 |
| 20 µM primer 2 | 13.75 |
| Total volume | 500 |

Into the above system was added 50 µL (180 ng) of the recovered products from the previous step. The mixture was mixed well and reaction was allowed under the conditions set out in Table 10.

**Table 10**

| Tempera ture | Time | Cycle |
|---|---|---|
| 95°C | 3 min | 1 |
| 95°C | 30 s | 7 |
| 56°C | 30 s | |
| 72°C | 4 min | |
| 68°C | 10 min | 1 |
| Ramping the temperature down to 4°C at a rate of 0.1°C/s | | |
| 4°C | Holding the temperature | - |

After reaction was complete, purification was conducted using 550 µL of Ampure XP magnetic beads, and the recovered products were dissolved with 80 µL of TE buffer. 1 µL of the recovered products was assayed with a Qubit dsDNA HS assay kit (Invitrogen Corp.) to quantitate the concentration of the products (Table 11). 2 µL of the products was used for reaction at the next step.

**Table 11**

| | Concentration (ng/µL) | Total amount (ng) | PCR amplification efficiency |
|---|---|---|---|
| Produc t 1 | 57.4 | 4592 | 1.59 |
| Produc t 2 | 52 | 4160 | 1.57 |

9. Removal of uracil: A reaction solution as shown in Table 12 below was formulated.

**Table 12**

| Reaction component | Volume (µL) |
|---|---|
| Enzyme-free pure water | 25.8 |
| 10× Taq buffer | 11 |
| USER enzyme (1 U/µL) | 13.2 |
| Total volume | 50 |

The above reaction solution was added into 60 µL (2 µg) of the reaction products from the previous step, and the mixture was mixed well and incubated at 37°C for 1 h.
10. Double strand cyclization:Reaction system 1 as shown in Table 13 below was formulated.

**Table 13**

| Reaction component | Volume (µL) |
|---|---|
| Enzyme-free pure water | 1520 |
| 10× TA buffer | 180 |
| Total volume | 1700 |

The reaction products from the previous step were added into reaction system 1. The mixture was mixed well and evenly dispensed into 4 tubes. The tubes were placed in a water bath at 50°C for reaction for 15 min. After the reaction was complete, the tubes were placed in a water bath at ambient temperature for reaction for 15 min.
Reaction system 2 as shown in Table 14 below was formulated.

**Table 14**

| Reaction component | Volume (µL) |
|---|---|
| Enzyme-free pure water | 98 |
| 20×Circ buffer | 100 |
| T4 DNA ligase (fast) (600 U/µL) | 2 |
| Total volume | 200 |

Into each of the 4 tubes of reaction system 1 was added 50 µL of reaction system 2, and the tubes were incubated at room temperature for 1 h.
330 µL of Ampure XP magnetic beads was added into the reaction products of each tube (500 µL). The mixture in each tube was mixed well and stood for 7-15 min. After placing the tubes on a magnetic rack, supernatant was collected. The supernatant was added with 170 µL of Ampure XP magnetic beads, and the mixture was mixed well and stood for 7-15 min. After placing the tubes on a magnetic rack, supernatant was aspirated off, and the magnetic beads were washed with 75% ethanol twice. After air drying the magnetic beads, 65 µL of TE buffer was added to each of the 4 tubes to dissolve the purified products.
11. Linear digestion: A reaction system as shown in Table 15 below was formulated.

**Table 15**

| Reaction component | Volume (µL) |
|---|---|
| Enzyme-free pure water | 0.7 |
| Plasmid-Safe 9X reaction buffer | 8.9 |
| Plasmid-Safe ATP-dependent DNase | 10.4 |
| Total volume | 20 |

The products from the previous step were added into the reaction system, and the mixture was mixed well and incubated at 37°C for 1 h.
Purification was conducted using 80 µL of Ampure XP magnetic beads, and the recovered products were dissolved using 82 µL of TE buffer. 1 µL of the recovered products were assayed with a Qubit dsDNA HS assay kit (Invitrogen Corp.) to quantitate the concentration of the products. 700 ng of the products was used for reaction at the next step. The initiation site for CNT reaction on the double-strand cyclized DNA formed in this example is in the form of gap.
12. Dephosphorylation:A reaction system as shown in Table 16 below was formulated.

**Table 16**

| Reaction component | Volume (µL) |
|---|---|
| Enzyme-free pure water | 39 |
| 10× NEBuffer 3 | 14 |
| Endo IV (10U/µL) | 7 |
| Total volume | 60 |

80 µL (700 ng) of the products from the previous step was added into the reaction system, and the mixture was mixed well and incubated at 37°C for 1 h.
Purification was conducted using 210 µL of Ampure XP magnetic beads, and the recovered products were dissolved using 55 µL of TE buffer. The concentration of the products was quantitated using a Qubit dsDNA HS assay kit (Invitrogen Corp.). 500 ng of the products were used for reaction at the next step.
13. Binding to streptavidin magnetic beads:Magnetic bead binding buffer and rinsing buffers were formulated according to the systems as shown in Tables 17-19.

**Table 17 2×LSBB (Low Salt Bead Binding Buffer)**

| Component | Content |
|---|---|
| Trihydroxymethyl aminomethane (pH 7.5) | 100 mM |
| Sodium chloride | 300 mM |
| Tween-20 | 0.005% |

**Table 18 LSWB (Low Salt Bead Wash Buffer)**

| Component | Content |
|---|---|
| Trihydroxymethyl aminomethane (pH 7.5) | 50 mM |
| Sodium chloride | 150 mM |
| Tween-20 | 0.05% |

**Table 19 HSWB (High Salt Bead Wash Buffer)**

| Component | Content |
|---|---|
| Trihydroxymethyl aminomethane (pH 7.5) | 50 mM |
| Sodium chloride | 500 mM |
| Tween-20 | 0.05% |

75 µL of MyOne Streptavidin C1 magnetic beads was used, and the supernatant was aspirated off. The beads were rinsed with 350 µL of 1×LSBB, then resuspended in 100 µL of 2×LSBB. 100 µL of dephosphorylated cyclized DNA (570 ng) diluted with enzyme-free pure water was added. The mixed solution of the beads and the DNA was incubated at 30 rpm at room temperature for 1 h.
The beads were rinsed with 350 µL of HSWB and 350 µL of LSWB respectively once, and equilibrated with 200 µL of 1×NEBuffer2 (containing 0.025% Tween-20). After each rinsing, the supernatant should be aspirated off, and after the last equilibration, the supernatant should be aspirated off thoroughly. The beads were placed on ice to precool for at least 10 min.
14. On-beads reaction for CNT: A reaction system as shown in Table 20 below was formulated.

**Table 20**

| Reaction component | Volume (µL) |
|---|---|
| Enzyme-free pure water | 51.2 |
| 10× NEBuffer2 | 6 |
| 2.5 mM dNTP (1:175) | 2 |
| PolI DNA polymerase (10 U/µL) | 0.8 |
| Total volume | 60 |

60 µL of the reaction system was quickly added into the precooled magnetic beads, and the mixture was rapidly mixed well and incubated at 8°C for 15 min.
1.5 µL of 0.5 M EDTA was added, and the mixture was mixed well. The beads were rinsed with 350 µL of HSWB and 350 µL of LSWB respectively once, and equilibrated with 200 µL of 1×NEBuffer4 (containing 0.025% Tween-20). The supernatant was aspirated off thoroughly.
15. On-beads reaction for 3'-5' exonuclease digestion:A reaction system as shown in Table 21 below was formulated.

**Table 21**

| Reaction component | Volume (µL) |
|---|---|
| Enzyme-free pure water | 65.5 |
| 10× NEBuffer4 | 8 |
| T7 Exo (10 U/µL) | 6.5 |
| Total volume | 80 |

80 µL of the reaction system was quickly added into the magnetic beads from the previous step, and the mixture was rapidly mixed well and incubated at 25°C for 1 h. 2 µL of 0.5 M EDTA was added, and the mixture was mixed well. The beads were rinsed with 350 µL of HSWB and 350 µL of LSWB respectively once, and equilibrated with 200 µL of 1×Exo VII reaction buffer (containing 0.025% Tween-20). The supernatant was aspirated off thoroughly.
16. On-beads reaction for single strand digestion:A reaction system as shown in Table 22 below was formulated.

**Table 22**

| Reaction component | Volume (µL) |
|---|---|
| Enzyme-free pure water | 27.8 |
| 5× ExoVII reaction buffer | 7 |
| Exo VII (10 U/µL) | 0.2 |
| Total volume | 35 |

35 µL of the reaction system was quickly added into the magnetic beads from the previous step, and the mixture was rapidly mixed well and incubated at 37°C for 30 min. 0.8 µL of 0.5 M EDTA was added, and the mixture was mixed well. The beads were rinsed with 350 µL of HSWB and 350 µL of LSWB respectively once, and equilibrated with 200 µL of 1×Exo VII reaction buffer (containing 0.025% Tween-20). The supernatant was aspirated off thoroughly.
17. On-beads reaction for end repairing:A reaction system as shown in Table 23 below was formulated.

**Table 23**

| Reaction component | Volume (µL) |
|---|---|
| Enzyme-free pure water | 69.2 |
| 10×NEBuffer2 | 8 |
| 25 mM dNTP | 0.8 |
| T4 deoxyribonucleic acid polymerase (3 U/µL) | 2 |
| Total volume | 80 |

80 µL of the reaction system was quickly added into the magnetic beads from the previous step, and the mixture was rapidly mixed well and incubated at 12°C for 20 min. 2 µL of 0.5 M EDTA was added, and the mixture was mixed well. The beads were rinsed with 350 µL of HSWB and 350 µL of LSWB respectively once, and equilibrated with 200 µL of 1×NEBuffer2 reaction buffer (containing 0.025% Tween-20). The supernatant was aspirated off thoroughly.
18. On-beads reaction for dephosphorylation: A reaction system as shown in Table 24 below was formulated.

**Table 24**

| Reaction component | Volume (µL) |
|---|---|
| Enzyme-free pure water | 48 |
| 10×NEBuffer2 | 6 |
| FastAp (1 U/µL) | 6 |
| Total volume | 60 |

60 µL of the reaction system was quickly added into the magnetic beads from the previous step, and the mixture was rapidly mixed well and incubated at 37°C for 45 min. 1.5 µL of 0.5 M EDTA was added, and the mixture was mixed well. The beads were rinsed with 350 µL of HSWB and 350 µL of LSWB respectively once, and equilibrated with 200 µL of 1×NEBuffer2 reaction buffer (containing 0.025% Tween-20). The supernatant was aspirated off thoroughly.
19. On-beads reaction for ligation of 5' adaptor B sequence:The adaptor B sequence used in this example was as follows:
5' adaptor B sequence: /5Phos/AAGTCGGAGGCCAAGCGTGCTTAGGA (SEQ ID NO: 6);
5' blocking sequence: GCCUCCGACT/ddT/ (SEQ ID NO: 7).

A mixed solution of 5' adaptor B (10 µM) was formulated according to the system as shown in Table 25.

**Table 25**

| Reaction component | Volume (µL) |
|---|---|
| 5' adaptor B sequence (100 µM) | 12 |
| 5' blocking sequence (100 µM) | 10 |
| TE buffer | 78 |
| Total | 100 |

A ligation reaction system was formulated according to the system as shown in Table 26.

**Table 26**

| Reaction component | Volume (µL) |
|---|---|
| Enzyme-free pure water | 43 |
| 3×ligation buffer 2 | 27 |
| 10 µM of the mixed solution of 5' adaptor B | 8 |
| Total | 78 |

78 µL of the reaction system was added into the magnetic beads from the previous step, and the mixture was mixed well. 2 µL of T4 DNA ligase (fast) (600 U/µL) were added rapidly, and the mixture was mixed well and incubated at 25°C for 60 min and at 65°C for 10 min, followed by cooling to 4°C. 1.5 µL of T4 polynueleotide kinase (10 U/µL) was added, and the mixture was mixed well and incubated at 37°C for 20 min. 2 µL of 0.5 M EDTA was added, and the mixture was mixed well. The beads were rinsed with 350 µL of HSWB and 350 µL of LSWB respectively once, and equilibrated with 200 µL of 1×NEBuffer2 reaction buffer (containing 0.025% Tween-20). The supernatant was aspirated off thoroughly.
20. On-beads reaction for ligation of 3' L-type adaptor B sequence:The 3' L-type adaptor B sequence used in this example was /5Phos/CATGTAGTGTACGATCCGACTT (SEQ ID NO: 8).
A 3' L-type adaptor reaction system was formulated according to the system as shown in Table 27:

**Table 27**

| Reaction component | Volume (µL) |
|---|---|
| Enzyme-free pure water | 44.5 |
| 3×ligation buffer 2 | 27 |
| L-type adaptor sequence (100 µM) | 6.5 |
| T4 DNA ligase (fast) (600 U/ µ L) | 2 |
| Total | 80 |

80 µL of the reaction system was added into the magnetic beads from the previous step, and the mixture was mixed well and incubated at 25°C for 60 min, followed by decreasing the temperature to 14°C at a rate of 0.1°C per second. 2 µL of 0.5 M EDTA was added, and the mixture was mixed well. The beads were rinsed with 350 µL of HSWB and 350 µL of LSWB respectively twice. The supernatant was aspirated off thoroughly.
40 µL of 0.1 M sodium hydroxide was added, and the mixture was mixed well and incubated at room temperature for 10 min. The supernatant was aspirated, and 20 µL of 0.3 M acidic buffer was added to neutralize the single-strand products separated, the total volume of the products after neutralization being 60 µL. This step achieved the ligation of the target nucleic acid fragments with adaptor B and obtained the target single-stranded DNA through alkali denaturation and separation.
21. Polymerase chain reaction:
Sequence of primer 1: /5Phos/CATGTAGTGTACGATCCGACTT (SEQ ID NO: 9);
Sequence of primer 2: TCCTAAGCACGCTTGGCCT (SEQ ID NO: 10).

A PCR system was formulated according to the system as shown in Table 28.

**Table 28**

| Reaction component | Volume (µL) |
|---|---|
| Enzyme-free pure water | 160 |
| 2×PfuTurbo Cx buffer | 219.8 |
| PfuTurbo Cx heat-activated nucleic acid polymerase (2.5 U/µL) | 8.2 |
| 20 µM of primer 1 | 11 |
| 20 µM of primer 2 | 11 |
| Total volume | 410 |

Into the above system was added 30 µL of the recovered products from the previous step, and the mixture was mixed well and allowed to react under the conditions as shown in Table 29.

**Table 29**

| Tempera ture | Time | Cycle |
|---|---|---|
| 95°C | 3 min | 1 |
| 95°C | 30 s | 7 |
| 56°C | 30 s | |
| 72°C | 4 min | |
| 68°C | 10 min | 1 |
| Ramping the temperature down to 4°C at a rate of 0.1°C/s | | |
| 4°C | Holding the temperature | - |

After reaction was complete, purification was conducted using 440 µL of Ampure XP magnetic beads, and the recovered product were dissolved with 80 µL of TE buffer. 1 µL of the recovered products was assayed with a Qubit dsDNA HS assay kit (Invitrogen Corp.) to quantitate the concentration of the products. 100 ng of the products was used for reaction at the next step. The size of the PCR product fragments was electrophoretically determined using an Agilent 2100 HS, the results being as shown in Fig. 7.
22. Single strand cyclization:
The mediating fragment has corresponding complementary sequences for ligating both ends of the single strand, the sequence of the mediating fragment being as follows: GTACACTACATGTCCTAAGCACGC (SEQ ID NO: 11).
10 µL of the mediating fragment (10 µM) was added into 100 ng of the PCR products from the previous step. The mixture was mixed well and incubated at 95°C for 3 min, followed by being rapidly placed on ice for cooling. A reaction system as shown in Table 30 below was formulated.

**Table 30**

| Reaction component | Volume (µL) |
|---|---|
| Enzyme-free water | 36.4 |
| 10× TA buffer (Epicentre Corp.) | 12 |
| 100 mM adenosine triphosphate | 1.2 |
| T4 Ligase (600 U/µL, Enzymatics) | 0.4 |
| Total | 50 |

50 µL of the reaction system was added into a mixed solution of the PCR products and the mediating fragment. The mixture was mixed well and incubated at 37°C for 60 min.
23. Digestion of linear DNA:A system as shown in Table 31 was formulated.

**Table 31**

| Reaction component | Volume (µL) |
|---|---|
| Enzyme-free water | 2 |
| 10× TA buffer (Epicentre Corp.) | 0.8 |
| Exonuclease 1 (20 U/µL, NEB Corp.) | 3.9 |
| Exonuclease 3 (100 U/µL, NEB Corp.) | 1.3 |
| Total | 8 |

8 µL of the reaction system was added into the ligation reaction solution from the previous step. The mixture was mixed well and incubated at 37°C for 60 min. 6 µL of 0.5 M EDTA was added and the mixture was mixed well. The products were purified and recovered using 170 µL of PEG32 magnetic beads, and redissolved in 55 µL of TE buffer.
The concentration and total amount of the final products in this example are as shown in Table 32.

**Table 32**

| | Concentration (ng/µL) | Total amount (ng) | Molecular weight (pmol) |
|---|---|---|---|
| Product 1 | 0.36 | 19.8 | 0.27 |
| Product 2 | 0.34 | 18.7 | 0.26 |

The results indicated that the concentration and total amount of the respective products met the requirement for subsequent sequencing (molecular weight ≧ 0.12 pmol). The adaptor B ligation products after PCR and before cyclization were determined using an Agilent 2100 capillary electrophoresis apparatus, the results being as shown in Fig. 7, the abscissa representing the size of base pairs, and the ordinate representing the strength of the fluorescent signal detected. The ordinate value of 0 represents the baseline signal of the detection. The higher the ordinate value, the stronger the fluorescent signal of the sample detected. In the figure, 35 and 1030 correspond to DNA standards of 35 bp and 1030 bp, which were the references for detecting the size of the sample fragments. In the figure, 218 and 219 represent the size of the main bands of product 1 and product 2, i.e., 218 bp and 219 bp respectively. The rectangular box on the right shows the distribution of the bands of the fragments to be detected, as simulated according to the detection signal. The results showed that the target bands obtained (as indicated by the arrows) clustered together, suggesting that the insert fragments could be effectively controlled in a very narrow range by means of CNT. Additionally, according to the size of the main band of the respective PCR products, the size of the adaptor A (54 bp) and the size of the adaptor B (48 bp), the size of the main band of the respective insert fragments could be calculated: 218-54-48=116bp=58bp*2 and 219-54-48=117bp≈59bp*2 respectively, demonstrating that a library of single-stranded cyclic nucleic acid fragments having double adaptors was successfully obtained in this example.

## Claims

1. A method for constructing a library of single-stranded cyclic nucleic acid fragments having double adaptors, comprising the following steps:
disrupting a nucleic acid into nucleic acid fragments for library construction;
ligating a first adaptor sequence to both ends of the nucleic acid fragments;
performing a first PCR amplification to obtain first products having the first adaptor sequence at both ends, wherein the primer sequences used in the first PCR have a uracil base site;
digesting the first products with a USER enzyme to form sticky ends, followed by cyclization to generate a gap; or alternatively, in the case that the primer sequences used in the first PCR also have a nickase recognition sequence, digesting the first products with a USER enzyme to form sticky ends, followed by cyclization, and then followed by digestion of the cyclized products with a nickase to generate a nick;
initiating controlled nick/gap translation reaction from the nick or gap by using the cyclic nucleic acid molecules as template, wherein the controlled nick/gap translation can move the gaps or nicks around the cyclic nucleic acid molecules, such that the gaps or nicks are located in the nucleic acid fragment and the length of the fragment can be controlled;
digesting and removing the portion of the respective cyclic nucleic acid molecules that does not undergo the controlled nick/gap translation reaction to obtain linear nucleic acid molecules, which comprises: first digesting the cyclic nucleic acid molecules with an enzyme having 5'-3' exonuclease activity until the gaps at both ends meet; and then digesting the resulting single strands with an enzyme having 3'-5' exonuclease activity or a single strand exonuclease;
ligating a second adaptor sequence to both ends of the linear nucleic acid molecules;
performing a second PCR amplification to obtain second products having the second adaptor sequence at both ends; and
denaturing the second products to obtain single-stranded nucleic acid molecules, and cyclizing one of the single-stranded nucleic acid molecules with a mediating sequence complementary to both ends of the single-stranded nucleic acid molecule to obtain the library of single-stranded cyclic nucleic acid fragments having double adaptors.

2. The method according to claim 1, wherein the first adaptor sequence comprises a first 5' adaptor sequence and a first 3' L-type adaptor sequence that respectively ligate to the 3' end and the 5' end of each strand of the fragments; the first 5' adaptor sequence comprises a 5'-end-phosphorylated long strand and a complementary short strand, the long strand having a tag sequence in the middle, the short strand having dideoxy modification at the 3' end, and the short strand comprising a uracil base site; and a portion of the first 3' L-type adaptor sequence which is adjacent to the ligated fragment is complementary to part of the bases of the first 5' adaptor sequence; and
said ligating a first adaptor sequence to both ends of the nucleic acid fragments specifically comprises:
dephosphorylating the nucleic acid fragments;
subjecting the dephosphorylated nucleic acid fragments to end repairing;
ligating the first 5' adaptor sequence to the 3' end of each strand of the nucleic acid fragments;
digesting the uracil base site of the short strand of the first 5' adaptor sequence with a USER enzyme;
phosphorylating the USER enzyme-digested nucleic acid fragments; and
ligating the first 3' L-type adaptor sequence to the 5' end of each strand of the phosphorylated nucleic acid fragments.

3. The method according to claim 1, wherein each of the primer sequences used in the first PCR has a nickase recognition sequence and a uracil base site; and after digesting the uracil base site with the USER enzyme, sticky ends are formed at both ends of the nucleic acid fragments, and cyclization occurs due to the complementarity of the sticky ends, generating cyclized nucleic acid molecules.

4. The method according to claim 1, wherein one of the primer sequences used in the first PCR has two uracil base sites, while the other primer sequence has a uracil base site; and after digesting the uracil base sites with the USER enzyme, sticky ends are formed at both ends of the nucleic acid fragments, and cyclization occurs due to the complementarity of the sticky ends, generating cyclized nucleic acid molecules.

5. The method according to claim 1, wherein following cyclizing the digested first products, the method further comprises:digesting the nucleic acid molecules that are not cyclized.

6. The method according to claim 1, wherein one of the primer sequences used in the first PCR harbors a biotin label; and prior to the controlled nick/gap translation reaction, streptavidin-labeled magnetic beads are used to bind the products from the first PCR, such that subsequent reactions are performed on the magnetic beads.

7. The method according to claim 1, wherein in the controlled nick/gap translation reaction, the length of the gap translation fragments generated is controlled by controlling at least one factor selected from the group consisting of the molar ratio of dNTPs to the nucleic acid molecules as template, the enzymatic reaction temperature and the enzymatic reaction time.

8. The method according to claim 1, wherein the second adaptor sequence comprises a second 5' adaptor sequence and a second 3' L-type adaptor sequence that respectively ligate to the 3' end and the 5' end of each strand of the linear nucleic acid molecules; the second 5' adaptor sequence comprises a 5-end-phosphorylated long strand and a complementary short strand, the short strand having dideoxy modification at the 3' end, and the short strand comprising a uracil base site; and a portion of the second 3' L-type adaptor sequence which is adjacent to the ligated fragment is complementary to part of the bases of the second 5' adaptor sequence; and
said ligating a second adaptor sequence to both ends of the linear nucleic acid molecules specifically comprises:
dephosphorylating the linear nucleic acid molecules;
subjecting the dephosphorylated linear nucleic acid molecules to end repairing;
ligating the second 5' adaptor sequence to the 3' end of each strand of the linear nucleic acid molecules;
digesting the uracil base site of the short strand of the second 5' adaptor sequence with a USER enzyme;
phosphorylating the USER-enzyme digested fragments; and
ligating the second 3' L-type adaptor sequence to the 5' end of each strand of the phosphorylated linear nucleic acid molecules.

9. The method according to claim 1, wherein following cyclizing the single-stranded nucleic acid molecules, the method further comprises:digesting the single-stranded nucleic acid molecules that are not cyclized.

10. A reagent for constructing a library of single-stranded cyclic nucleic acid fragments having double adaptors, comprising the following components:
a first adaptor sequence, which comprises a first 5' adaptor sequence and a first 3' L-type adaptor sequence that respectively ligate to the 3' end and the 5' end of each strand of the fragments, wherein the first 5' adaptor sequence comprises a 5'-end-phosphorylated long strand and a complementary short strand, the long strand having a tag sequence in the middle, the short strand having dideoxy modification at the 3' end, and the short strand comprising a uracil base site; and a portion of the first 3' L-type adaptor sequence which is adjacent to the ligated fragment is complementary to part of the bases of the first 5' adaptor sequence;
primers for a first PCR, which have uracil base sites or have a nickase recognition sequence and a uracil base site, and which are used to obtain first products having the first adaptor sequence at both ends by the first PCR amplification;
a nickase, which is used for digesting the first products to generate a nick;
a USER enzyme, which is used for digesting the first products to generate sticky ends and gaps for cyclization;
components for gap translation reaction, which are used for initiating controlled nick/gap translation reaction from the nick or gap by using the cyclic nucleic acid molecules as template, wherein the controlled nick/gap translation can move the gaps or nicks around the cyclic nucleic acid molecules, such that the gaps or nicks are located in the nucleic acid fragment and the length of the fragment can be controlled;
digestive enzymes comprising an enzyme having 5'-3' exonuclease activity and either an enzyme having 3'-5' exonuclease activity or a single strand exonuclease, which are used for: first digesting the cyclic nucleic acid molecules with an enzyme having 5'-3' exonuclease activity until the gaps at both ends meet; and then digesting the resulting single strands with an enzyme having 3'-5' exonuclease activity or a single strand exonuclease;
a second adaptor sequence, which comprises a second 5' adaptor sequence and a second 3' L-type adaptor sequence that respectively ligate to the 3' end and the 5' end of each strand of the linear nucleic acid molecules; wherein the second 5' adaptor sequence comprises a 5-end-phosphorylated long strand and a complementary short strand, the short strand having dideoxy modification at the 3' end, and the short strand comprising a uracil base site; and a portion of the second 3' L-type adaptor sequence which is adjacent to the ligated fragment is complementary to part of the bases of the second 5' adaptor sequence;
primers for a second PCR, which are used for performing a second PCR amplification to obtain second products having the second adaptor sequence at both ends; and
a mediating sequence, which is complementary to both ends of one of the single-stranded nucleic acid molecules obtained following denaturation of the second products, and which is used for cyclizing the single-stranded nucleic acid molecule to obtain the library of single-stranded cyclic nucleic acid fragments having double adaptors.

## Patentansprüche

1. Ein Verfahren zur Konstruktion einer Bibliothek von einzelsträngigen zyklischen Nukleinsäurefragmenten mit Doppeladaptern, das folgende Schritte beinhaltet:
Aufbrechen einer Nukleinsäure in Nukleinsäurefragmente zur Konstruktion einer Bibliothek;
Ligieren einer ersten Adaptersequenz an beide Enden der Nukleinsäurefragmente;
Durchführen einer ersten PCR-Amplifikation, um erste Produkte zu erhalten, welche an beiden Enden die erste Adaptersequenz aufweisen, wobei die in der ersten PCR verwendeten Primersequenzen eine Uracil-Basenstelle aufweisen;
Aufschließen der ersten Produkte mit einem USER-Enzym, um klebrige Enden zu bilden, gefolgt von Zyklisierung, um eine Lücke zu erzeugen; oder alternativ, in dem Fall, dass die in der ersten PCR verwendeten Primersequenzen auch eine Nickase-Erkennungssequenz aufweisen, Aufschließen der ersten Produkte mit einem USER-Enzym, um klebrige Enden zu bilden, gefolgt von Zyklisierung, und anschließend gefolgt von dem Aufschluss der zyklisierten Produkte mit einer Nickase, um einen Schnitt zu erzeugen;
Einleiten einer gesteuerten Schnitt/Lücken-Translationsreaktion von dem Schnitt oder der Lücke durch Verwenden der zyklischen Nukleinsäuremoleküle als Matrize, wobei die gesteuerte Schnitt/Lücken-Translation die Lücken oder Schnitte um die zyklischen Nukleinsäuremoleküle herum bewegen kann, sodass die Lücken oder Schnitte sich in dem Nukleinsäurefragment befinden und die Länge des Fragments gesteuert werden kann;
Aufschließen und Entfernen des Abschnitts der jeweiligen zyklischen Nukleinsäuremoleküle, der die gesteuerte Schnitt/Lücken-Translationsreaktion nicht durchläuft, um lineare Nukleinsäuremoleküle zu erhalten, was Folgendes beinhaltet: erstes Aufschließen der zyklischen Nukleinsäuremoleküle mit einem Enzym mit 5'-3'-Exonuklease-Aktivität, bis die Lücken an beiden Enden aufeinandertreffen; und danach Aufschließen der resultierenden Einzelstränge mit einem Enzym mit 3'-5'-Exonucleaseaktivität oder einer Einzelstrang-Exonuclease;
Ligieren einer zweiten Adaptersequenz an beide Enden der linearen Nukleinsäuremoleküle;
Durchführen einer zweiten PCR-Amplifikation, um zweite Produkte zu erhalten, die an beiden Enden die zweite Adaptersequenz aufweisen; und
Denaturieren der zweiten Produkte, um einzelsträngige Nukleinsäuremoleküle zu erhalten, und Zyklisieren eines der einzelsträngigen Nukleinsäuremoleküle mit einer Mediatorsequenz, die zu beiden Enden des einzelsträngigen Nukleinsäuremoleküls komplementär ist, um die Bibliothek von einzelsträngigen zyklischen Nukleinsäurefragmenten mit Doppeladaptern zu erhalten.

2. Verfahren nach Anspruch 1, wobei die erste Adaptersequenz eine erste 5'-Adaptersequenz und eine erste 3'-L-Typ-Adaptersequenz beinhaltet, die an das 3'-Ende bzw. das 5'-Ende jedes Strangs der Fragmente ligieren; wobei die erste 5'-Adaptersequenz einen am 5'-Ende phosphorylierten langen Strang und einen komplementären kurzen Strang beinhaltet, wobei der lange Strang eine Tag-Sequenz in der Mitte aufweist, der kurze Strang eine Dideoxy-Modifikation am 3'-Ende aufweist und der kurze Strang eine Uracil-Basenstelle beinhaltet; und ein Abschnitt der ersten 3'-L-Typ-Adaptersequenz, die an das ligierte Fragment angrenzt, komplementär zu einem Teil der Basen der ersten 5'-Adaptersequenz ist; und
das genannte Ligieren einer ersten Adaptersequenz an beide Enden der Nukleinsäurefragmente spezifisch Folgendes beinhaltet:
Dephosphorylieren der Nukleinsäurefragmente;
Unterziehen der dephosphorylierten Nukleinsäurefragmente einer Endreparatur;
Ligieren der ersten 5'-Adaptersequenz an das 3'-Ende jedes Strangs der Nukleinsäurefragmente;
Aufschließen der Uracil-Basenstelle des kurzen Strangs der ersten 5'-Adaptersequenz mit einem USER-Enzym;
Phosphorylieren der mit dem USER-Enzym aufgeschlossenen Nukleinsäurefragmente; und
Ligieren der ersten 3'-L-Typ-Adaptersequenz an das 5'-Ende jedes Strangs der phosphorylierten Nukleinsäurefragmente.

3. Verfahren nach Anspruch 1, wobei jede von den in der ersten PCR verwendeten Primersequenzen eine Nickase-Erkennungssequenz und eine Uracil-Basenstelle aufweist; und wobei nach dem Aufschließen der Uracil-Basenstelle mit dem USER-Enzym an beiden Enden der Nukleinsäurefragmente klebrige Enden gebildet werden und es aufgrund der Komplementarität der klebrigen Enden zur Zyklisierung kommt, wodurch zyklisierte Nukleinsäuremoleküle erzeugt werden.

4. Verfahren nach Anspruch 1, wobei eine der in der ersten PCR verwendeten Primersequenzen zwei Uracil-Basenstellen aufweist, während die andere Primersequenz eine Uracil-Basenstelle aufweist; und wobei nach dem Aufschließen der Uracil-Basenstellen mit dem USER-Enzym an beiden Enden der Nukleinsäurefragmente klebrige Enden gebildet werden und es aufgrund der Komplementarität der klebrigen Enden zur Zyklisierung kommt, wodurch zyklisierte Nukleinsäuremoleküle erzeugt werden.

5. Verfahren nach Anspruch 1, wobei im Anschluss an das Zyklisieren der aufgeschlossenen ersten Produkte das Verfahren ferner Folgendes beinhaltet: Aufschließen der nicht zyklisierten Nukleinsäuremoleküle.

6. Verfahren nach Anspruch 1, wobei eine der in der ersten PCR verwendeten Primersequenzen eine Biotinmarkierung birgt; und wobei vor der gesteuerten Schnitt/Lücken-Translationsreaktion streptavidinmarkierte magnetische Beads zum Binden der Produkte von der ersten PCR verwendet werden, sodass anschließende Reaktionen an den magnetischen Beads ausgeführt werden.

7. Verfahren nach Anspruch 1, wobei in der gesteuerten Schnitt/Lücken-Translationsreaktion die Länge der erzeugten Lücken-Translationsfragmente gesteuert wird, indem mindestens ein Faktor gesteuert wird, ausgewählt aus der Gruppe, bestehend dem Molverhältnis der dNTPs zu den Nukleinsäuremolekülen als Matrize, der Temperatur der enzymatischen Reaktion und der Zeit der enzymatischen Reaktion.

8. Verfahren nach Anspruch 1, wobei die zweite Adaptersequenz eine zweite 5'-Adaptersequenz und eine zweite 3'-L-Typ-Adaptersequenz beinhaltet, die an das 3'-Ende bzw. an das 5'-Ende jedes Strangs der linearen Nukleinsäuremoleküle ligieren; wobei die zweite 5'-Adaptersequenz einen am 5-Ende phosphorylierten langen Strang und einen komplementären kurzen Strang beinhaltet, wobei der kurze Strang eine Dideoxy-Modifikation am 3'-Ende aufweist und der kurze Strang eine Uracil-Basenstelle beinhaltet; und ein Abschnitt der zweiten 3'-L-Typ-Adaptersequenz, die an das ligierte Fragment angrenzt, komplementär zu einem Teil der Basen der zweiten 5'-Adaptersequenz ist; und
das genannte Ligieren einer zweiten Adaptersequenz an beide Enden der linearen Nukleinsäuremoleküle spezifisch Folgendes beinhaltet:
Dephosphorylieren der linearen Nukleinsäuremoleküle;
Unterziehen der dephosphorylierten linearen Nukleinsäuremoleküle einer Endreparatur;
Ligieren der zweiten 5'-Adaptersequenz an das 3'-Ende jedes Strangs der linearen Nukleinsäuremoleküle;
Aufschließen der Uracil-Basenstelle des kurzen Strangs der zweiten 5'-Adaptersequenz mit einem USER-Enzym;
Phosphorylieren der mit dem USER-Enzym aufgeschlossenen Fragmente; und
Ligieren der zweiten 3'-L-Typ-Adaptersequenz an das 5'-Ende jedes Strangs der phosphorylierten linearen Nukleinsäurefragmente.

9. Verfahren nach Anspruch 1, wobei im Anschluss an das Zyklisieren der einzelsträngigen Nukleinsäuremoleküle das Verfahren ferner Folgendes beinhaltet: Aufschließen der nicht zyklisierten einzelsträngigen Nukleinsäuremoleküle.

10. Ein Reagens zur Konstruktion einer Bibliothek von einzelsträngigen zyklischen Nukleinsäurefragmenten mit Doppeladaptern, beinhaltend folgende Komponenten:
eine erste Adaptersequenz, die eine erste 5'-Adaptersequenz und eine erste 3'-L-Typ-Adaptersequenz, die an das 3'-Ende bzw. an das 5'-Ende jedes Strangs der Fragmente ligieren, wobei die erste 5'-Adaptersequenz einen am 5'-Ende phosphorylierten langen Strang und einen komplementären kurzen Strang beinhaltet, wobei der lange Strang eine Tag-Sequenz in der Mitte aufweist, der kurze Strang eine Dideoxy-Modifikation am 3'-Ende aufweist und der kurze Strang eine Uracil-Basenstelle beinhaltet; und wobei ein Abschnitt der ersten 3'-L-Typ-Adaptersequenz, die an das ligierte Fragment angrenzt, komplementär zu einem Teil der Basen der ersten 5'-Adaptersequenz ist;
Primer für eine erste PCR, die Uracil-Basenstellen aufweisen oder eine Nickase-Erkennungssequenz und eine Uracil-Basenstelle aufweisen und die verwendet werden, um erste Produkte, die an beiden Enden die Adaptersequenz aufweisen, durch die erste PCR-Amplifikation zu erhalten;
eine Nickase, die zum Aufschließen der ersten Produkte verwendet wird, um einen Schnitt zu erzeugen;
ein USER-Enzym, das zum Aufschließen der ersten Produkte verwendet wird, um klebrige Enden und Lücken für die Zyklisierung zu erzeugen;
Komponenten für die Lücken-Translationsreaktion, die zum Einleiten einer gesteuerten Schnitt/Lücken-Translationsreaktion von dem Schnitt oder der Lücke durch Verwenden der zyklischen Nukleinsäuremoleküle als Matrize verwendet werden, wobei die gesteuerte Schnitt/Lücken-Translation die Lücken oder Schnitte um die zyklischen Nukleinsäuremoleküle herum bewegen kann, sodass die Lücken oder Schnitte sich in dem Nukleinsäurefragment befinden und die Länge des Fragments gesteuert werden kann;
aufschließende Enzyme, die ein Enzym mit 5'-3'-Exonuklease-Aktivität und entweder ein Enzym mit 3'-5'-Exonuklease-Aktivität oder einer Einzelstrang-Exonuclease beinhalten, die für Folgendes verwendet werden: erstes Aufschließen der zyklischen Nukleinsäuremoleküle mit einem Enzym mit 5'-3'-Exonuclease-Aktivität, bis die Lücken an beiden Enden aufeinandertreffen; und danach Aufschließen der resultierenden Einzelstränge mit einem Enzym mit 3'-5'-Exonucleaseaktivität oder einer Einzelstrang-Exonuclease;
eine zweite Adaptersequenz, die eine zweite 5'-Adaptersequenz und eine zweite 3'-L-Typ-Adaptersequenz beinhalten, die an das 3'-Ende bzw. an das 5'-Ende jedes Strangs der linearen Nukleinsäuremoleküle ligieren; wobei die zweite 5'-Adaptersequenz einen am 5-Ende phosphorylierten langen Strang und einen komplementären kurzen Strang beinhaltet, der kurze Strang eine Dideoxy-Modifikation am 3'-Ende aufweist und der kurze Strang eine Uracil-Basenstelle beinhaltet; und ein Abschnitt der zweiten 3'-L-Typ-Adaptersequenz, die an das ligierte Fragment angrenzt, komplementär zu einem Teil der Basen der zweiten 5'-Adaptersequenz ist;
Primer für eine zweite PCR, die zum Durchführen einer zweiten PCR-Amplifikation verwendet werden, um zweite Produkte zu erhalten, die an beiden Enden die zweite Adaptersequenz aufweisen; und
eine Mediatorsequenz, die zu beiden Enden von einem der einzelsträngigen Nukleinsäuremoleküle komplementär ist, die im Anschluss an die Denaturierung der zweiten Produkte erhalten werden, und die zum Zyklisieren des einzelsträngigen Nukleinsäuremoleküls verwendet wird, um die Bibliothek von einzelsträngigen zyklischen Nukleinsäurefragmenten mit Doppeladaptern zu erhalten.

## Revendications

1. Procédé destiné à construire une banque de fragments d'acide nucléique cycliques simple brin ayant des adaptateurs doubles, comprenant les étapes suivantes :
la perturbation d'un acide nucléique en fragments d'acide nucléique pour la construction d'une banque ;
la ligature d'une première séquence d'adaptateur aux deux extrémités des fragments d'acide nucléique ;
la réalisation d'une première amplification par PCR pour obtenir des premiers produits ayant la première séquence d'adaptateur au niveau des deux extrémités, dans lequel les séquences d'amorce utilisées dans la première PCR ont un site de base uracile ;
la digestion des premiers produits avec une enzyme USER pour former des extrémités cohésives, suivie par la cyclisation pour générer un espace ; ou en variante, dans le cas où les séquences d'amorce utilisées dans la première PCR ont aussi une séquence de reconnaissance d'entaillase, la digestion des premiers produits avec une enzyme USER pour former des extrémités cohésives, suivie par une cyclisation, et puis suivie par la digestion des produits cyclisés avec une entaillase pour générer une entaille ;
l'induction d'une réaction de traduction entaille/espace contrôlée à partir de l'entaille ou de l'espace en utilisant les molécules d'acide nucléique cycliques comme matrice, dans lequel la traduction entaille/espace contrôlée peut déplacer les espaces ou les entailles autour des molécules d'acide nucléique cycliques, de sorte que les espaces ou les entailles sont situés dans le fragment d'acide nucléique et la longueur du fragment peut être contrôlée ;
la digestion et l'élimination de la partie des molécules d'acide nucléique cycliques respectives qui ne subit pas la réaction de traduction cassure/espace contrôlée pour obtenir des molécules d'acide nucléique linéaires, qui comprend : en premier la digestion des molécules d'acide nucléique cycliques avec une enzyme ayant une activité d'exonucléase de 5' à 3' jusqu'à ce que les espaces au niveau des deux extrémités se rencontrent ; et puis la digestion des simples brins qui en résultent avec une enzyme ayant une activité d'exonucléase de 3' à 5' ou une exonucléase simple brin ;
la ligature d'une deuxième séquence d'adaptateur aux deux extrémités des molécules d'acide nucléique linéaires ;
la réalisation d'une deuxième amplification par PCR pour obtenir des deuxièmes produits ayant la deuxième séquence d'adaptateur au niveau des deux extrémités ; et
la dénaturation des deuxièmes produits pour obtenir des molécules d'acide nucléique simple brin, et la cyclisation de l'une des molécules d'acide nucléique simple brin avec une séquence de médiation complémentaire aux deux extrémités de la molécule d'acide nucléique simple brin pour obtenir la banque de fragments d'acide nucléique cycliques simple brin ayant des adaptateurs doubles.

2. Procédé selon la revendication 1, dans lequel la première séquence d'adaptateur comprend une première séquence d'adaptateur en 5' et une première séquence d'adaptateur de type L en 3' qui se ligaturent respectivement à l'extrémité 3' et à l'extrémité 5' de chaque brin des fragments ; la première séquence d'adaptateur en 5' comprend un brin long phosphorylé à l'extrémité 5' et un brin court complémentaire, le brin long ayant une séquence de marquage au milieu, le brin court ayant une modification didésoxy au niveau de l'extrémité 3', et le brin court comprenant un site de base uracile ; et une partie de la première séquence d'adaptateur de type L en 3' qui est adjacente au fragment ligaturé est complémentaire à une partie des bases de la première séquence d'adaptateur en 5' ; et
ladite ligature d'une première séquence d'adaptateur aux deux extrémités des fragments d'acide nucléique comprend spécifiquement :
la déphosphorylation des fragments d'acide nucléique ;
le fait de soumettre les fragments d'acide nucléique déphosphorylés à une réparation d'extrémités ;
la ligature de la première séquence d'adaptateur en 5' à l'extrémité 3' de chaque brin des fragments d'acide nucléique ;
la digestion du site de base uracile du brin court de la première séquence d'adaptateur en 5' avec une enzyme USER ;
la phosphorylation des fragments d'acide nucléique digérés par l'enzyme USER ; et
la ligature de la première séquence d'adaptateur de type L en 3' à l'extrémité 5' de chaque brin des fragments d'acide nucléique phosphorylés.

3. Procédé selon la revendication 1, dans lequel chacune des séquences d'amorce utilisées dans la première PCR a une séquence de reconnaissance d'entaillase et un site de base uracile ; et après la digestion du site de base uracile avec l'enzyme USER, des extrémités cohésives sont formées au niveau des deux extrémités des fragments d'acide nucléique, et la cyclisation a lieu en raison de la complémentarité des extrémités cohésives, générant des molécules d'acide nucléique cyclisées.

4. Procédé selon la revendication 1, dans lequel l'une des séquences d'amorce utilisées dans la première PCR a deux sites de base uracile, alors que l'autre séquence d'amorce a un site de base uracile ; et après la digestion des sites de base uracile avec l'enzyme USER, des extrémités cohésives sont formées au niveau des deux extrémités des fragments d'acide nucléique, et la cyclisation a lieu en raison de la complémentarité des extrémités cohésives, générant des molécules d'acide nucléique cyclisées.

5. Procédé selon la revendication 1, dans lequel suite à la cyclisation des premiers produits digérés, le procédé comprend en outre : la digestion des molécules d'acide nucléique qui ne sont pas cyclisées.

6. Procédé selon la revendication 1, dans lequel l'une des séquences d'amorce utilisées dans la première PCR abrite un marqueur à base de biotine ; et avant la réaction de traduction entaille/espace contrôlée, des billes magnétiques marquées à la streptavidine sont utilisées pour lier les produits de la première PCR, de sorte que les réactions subséquentes sont réalisées sur les billes magnétiques.

7. Procédé selon la revendication 1, dans lequel dans la réaction de traduction entaille/espace contrôlée, la longueur des fragments de traduction d'espaces générés est contrôlée en contrôlant au moins un facteur sélectionné parmi le groupe constitué du rapport molaire des dNTP et des molécules d'acide nucléique comme matrice, de la température de la réaction enzymatique et du temps de la réaction enzymatique.

8. Procédé selon la revendication 1, dans lequel la deuxième séquence d'adaptateur comprend une deuxième séquence d'adaptateur en 5' et une deuxième séquence d'adaptateur de type L en 3' qui se ligaturent respectivement à l'extrémité 3' et à l'extrémité 5' de chaque brin des molécules d'acide nucléique linéaires ; la deuxième séquence d'adaptateur en 5' comprend un brin long phosphorylé à l'extrémité 5 et un brin court complémentaire, le brin court ayant une modification didésoxy au niveau de l'extrémité 3', et le brin court comprenant un site de base uracile ; et une partie de la deuxième séquence d'adaptateur de type L en 3' qui est adjacente au fragment ligaturé est complémentaire à la partie des bases de la deuxième séquence d'adaptateur en 5' ; et
ladite ligature d'une deuxième séquence d'adaptateur au niveau des deux extrémités des molécules d'acide nucléique linéaires comprend spécifiquement :
la déphosphorylation des molécules d'acide nucléique linéaires ;
le fait de soumettre les molécules d'acide nucléique linéaires déphosphorylées à une réparation d'extrémités ;
la ligature de la deuxième séquence d'adaptateur en 5' à l'extrémité 3' de chaque brin des molécules d'acide nucléique linéaires ;
la digestion du site de base uracile du brin court de la deuxième séquence d'adaptateur en 5' avec une enzyme USER ;
la phosphorylation des fragments digérés avec une enzyme USER ; et
la ligature de la deuxième séquence d'adaptateur de type L en 3' à l'extrémité 5' de chaque brin des molécules d'acide nucléique linéaires phosphorylées.

9. Procédé selon la revendication 1, dans lequel suite à la cyclisation des molécules d'acide nucléique simple brin, le procédé comprend en outre : la digestion des molécules d'acide nucléique simple brin qui ne sont pas cyclisées.

10. Réactif destiné à construire une banque de fragments d'acide nucléique cycliques simple brin ayant des adaptateurs doubles, comprenant les composants suivants :
une première séquence d'adaptateur, qui comprend une première séquence d'adaptateur en 5' et une première séquence d'adaptateur de type L en 3' qui se ligaturent respectivement à l'extrémité 3' et à l'extrémité 5' de chaque brin des fragments, dans lequel la première séquence d'adaptateur en 5' comprend un brin long phosphorylé à l'extrémité 5' et un brin court complémentaire, le brin long ayant une séquence de marquage au milieu, le brin court ayant une modification didésoxy au niveau de l'extrémité 3', et le brin court comprenant un site de base uracile ; et une partie de la première séquence d'adaptateur de type L en 3' qui est adjacente au fragment ligaturé est complémentaire à la partie des bases de la première séquence d'adaptateur en 5' ;
des amorces pour une première PCR, qui ont des sites de base uracile ou ont une séquence de reconnaissance d'entaillase et un site de base uracyle, et qui sont utilisées pour obtenir des premiers produits ayant la première séquence d'adaptateur aux deux extrémités par la première amplification par PCR ;
une entaillase, qui est utilisée pour digérer les premiers produits pour générer une entaille ;
une enzyme USER, qui est utilisée pour digérer les premiers produits pour générer des extrémités cohésives et des espaces pour la cyclisation ;
des composants pour la réaction de traduction d'espaces, qui sont utilisés pour induire la réaction de traduction entaille/espace contrôlée à partir de l'entaille ou de l'espace en utilisant les molécules d'acide nucléique cycliques comme matrice, dans lequel la traduction entaille/espace contrôlée peut déplacer les espaces ou les entailles autour des molécules d'acide nucléique cycliques, de sorte que les espaces ou les entailles sont situés dans le fragment d'acide nucléique et la longueur du fragment peut être contrôlée ;
des enzymes digestives comprenant une enzyme ayant une activité exonucléase de 5' à 3' et soit une enzyme ayant une activité exonucléase de 3' à 5' ou une exonucléase simple brin, qui sont utilisées pour : en premier la digestion des molécules d'acide nucléique cycliques avec une enzyme ayant une activité exonucléase de 5' à 3' jusqu'à ce que les espaces au niveau des deux extrémités se rencontrent ; et puis la digestion des simples brins qui en résultent avec une enzyme ayant une activité d'exonucléase de 3' à 5' ou une exonucléase simple brin ;
une deuxième séquence d'adaptateur, qui comprend une deuxième séquence d'adaptateur en 5' et une deuxième séquence d'adaptateur de type L en 3' qui se ligaturent respectivement à l'extrémité 3' et à l'extrémité 5' de chaque brin des molécules d'acide nucléique linéaires ; dans lequel la deuxième séquence d'adaptateur en 5' comprend un brin long phosphorylé à l'extrémité 5 et un brin court complémentaire, le brin court ayant une modification didésoxy au niveau de l'extrémité 3', et le brin court comprenant un site de base uracile ; et une partie de la deuxième séquence de type L en 3' qui est adjacente au fragment ligaturé est complémentaire à une partie des bases de la deuxième séquence d'adaptateur en 5' ;
des amorces pour une deuxième PCR, qui sont utilisées pour réaliser une deuxième amplification par PCR pour obtenir des deuxièmes produits ayant la deuxième séquence d'adaptateur au niveau des deux extrémités ; et
une séquence de médiation, qui est complémentaire aux deux extrémités de l'une des molécules d'acide nucléique simple brin obtenues suite à la dénaturation des deuxièmes produits, et qui est utilisée pour la cyclisation de la molécule d'acide nucléique simple brin pour obtenir la banque de fragments d'acide nucléique cycliques simple brin ayant des adaptateurs doubles.


### SEQUENCE LISTING

<110> BGI SHENZHEN£¬BGI SHENZHEN CO., LIMITED
<120> METHOD AND REAGENT FOR CONSTRUCTING NUCLEIC ACID DOUBLE-LINKER SINGLESTRAND
   CYCLICAL LIBRARY
<130> 14P20694
<160> 11
<170> PatentIn version 3.3
<210> 1
   <211> 46
   <212> DNA
   <213> Artificial sequence
<220>
   <221> phosphorylation modification
   <222> (1)..(1)
<220>
   <221> dideoxy modification
   <222> (46)..(46)
<400> 1
   actgctgacg tactgtgtca taaatagcac gagacgttct cgactc 46
<210> 2
   <211> 12
   <212> DNA
   <213> Artificial sequence
<220>
   <221> Uracil
   <222> (5)..(5)
<220>
   <221> dideoxy modification
   <222> (12)..(12)
<400> 2
   tacgtcagca gt 12
<210> 3
   <211> 18
   <212> DNA
   <213> Artificial sequence
<220>
   <221> Uracil
   <222> (11)..(11)
<400> 3
   cgttctcgac tcagcagt 18
<210> 4
   <211> 43
   <212> DNA
   <213> Artificial sequence
<220>
   <221> Uracil
   <222> (11)..(11)
<220>
   <221> biotin modification
   <222> (16)..(16)
<220>
   <221> Uracil
   <222> (37)..(37)
<400> 4
   gtcgagaacg tctcgtgcta tttatgacac agtacgtcag cag 43
<210> 5
   <211> 18
   <212> DNA
   <213> Artificial sequence
<220>
   <221> Uracil
   <222> (12)..(12)
<400> 5
   acgttctcga ctcagcag 18
<210> 6
   <211> 26
   <212> DNA
   <213> Artificial sequence
<220>
   <221> phosphorylation modification
   <222> (1)..(1)
<400> 6
   aagtcggagg ccaagcgtgc ttagga 26
<210> 7
   <211> 11
   <212> DNA
   <213> Artificial sequence
<220>
   <221> Uracil
   <222> (4) .. (4)
<220>
   <221> dideoxy modification
   <222> (11)..(11)
<400> 7
   gcctccgact t 11
<210> 8
   <211> 22
   <212> DNA
   <213> Artificial sequence
<220>
   <221> phosphorylation modification
   <222> (1)..(1)
<400> 8
   catgtagtgt acgatccgac tt 22
<210> 9
   <211> 22
   <212> DNA
   <213> Artificial sequence
<220>
   <221> phosphorylation modification
   <222> (1)..(1)
<400> 9
   catgtagtgt acgatccgac tt 22
<210> 10
   <211> 19
   <212> DNA
   <213> Artificial sequence
<400> 10
   tcctaagcac gcttggcct 19
<210> 11
   <211> 24
   <212> DNA
   <213> Artificial sequence
<400> 11
   gtacactaca tgtcctaagc acgc 24